Europäisches Patentamt

European Patent Office

Office européen des brevets

(11) Publication number: **0 277 366**
**A1**

## (12) EUROPEAN PATENT APPLICATION

(21) Application number: **87119364.5**

(51) Int. Cl.⁴: **A61M 25/00**

(22) Date of filing: **30.12.87**

<table>
<tr>
<td>

The title of the invention has been amended (Guidelines for Examination in the EPO, A-III, 7.3).

(30) Priority: **06.01.87 US 654**

(43) Date of publication of application:
**10.08.88 Bulletin 88/32**

(84) Designated Contracting States:
**CH DE FR GB IT LI NL**

</td>
<td>

(71) Applicant: **ADVANCED CARDIOVASCULAR SYSTEMS, INC.**
**1395 Charleston Road**
**Mountain View, CA 94039-7101(US)**

(72) Inventor: **Wand, Bruce H.**
**4193 Mystic Court**
**San José, California 95124(US)**
Inventor: **Brown, Peter S.**
**742 A Mora Drive**
**Los Altos Hills, California 94022(US)**
Inventor: **Samson, Wilfried J.**
**19691 Farwell Avenue**
**Saratoga, California 95070(US)**

(74) Representative: **Baillie, Iain Cameron et al**
**c/o Ladas & Parry Isartorplatz 5**
**D-8000 München 2(DE)**

</td>
</tr>
</table>

(54) **Guiding catheter assembly and method for making it.**

(57) Guiding catheter assembly (11) adapted to be inserted into a vessel having a first guiding catheter (12) and a second guiding catheter (13) removably disposed within the first guiding catheter (12). The second guiding catheter (13) has a length so that it extends beyond the distal extremity of the first guiding catheter (13). Each of the first and second guiding catheters (12, 13) includes a flexible elongate tubular member (16, 18) having a bore (17, 19) extending therethrough and a fitting (31) secured to the proximal extremity of the tubular member (16, 18). The bore (17, 19) in the tubular member (16, 18) is of a size so that the portion of the second guiding catheter (13) extending beyond the fitting (31) can be introduced into the bore (17) of the tubular member (16) of the first guiding catheter (12).

FIG. — 1

# GUIDING CATHETER ASSEMBLY AND METHOD

## Technical Field

This invention relates to a guiding catheter assembly and method and more particularly to a guiding catheter assembly and method which includes a subselective guiding catheter.

## Background of the Invention

Guiding catheters have heretofore been provided, for example, of the type disclosed in U.S. Patent No. 4,323,071. It has been found that for certain applications, such guiding catheters have too great a wall thickness so that an inner bore of the desired diameter could not be produced. In addition, such catheters in certain applications have not had the desired torqueability. It is therefore desirable to provide a new and improved guiding catheter and method for making the same.

In general, it is an object of the present invention to provide a guiding catheter assembly and method in which the guiding catheter utilized therein have increased bore sizes without increasing the corresponding outer diameters of the guiding catheters.

Another object of the invention is to provide a guiding catheter assembly of the above character which has improved torqueability.

Another object of the invention is to provide a guiding catheter assembly of the above character which makes the possible use of a subselective catheter.

Another object of the invention is to provide a guiding catheter assembly which has good column strength even though it has a thinner wall section.

Additional objects and features of the invention will appear from the following description wherein the preferred embodiment is set forth in detail in conjunction with the accompanying drawings.

## The Drawings

FIGURE 1 is a side elevation on the above guiding catheter assembly incorporating the present invention showing an outer guiding catheter with an inner or subselective catheter disposed therein.

FIGURE 2 is a side elevational view of the proximal portion of the assembly shown in Figure 1 but showing the subselective catheter only partially inserted into the outer guiding catheter.

FIGURE 3 is a side elevational view of the first or outer guiding catheter utilized in the assembly shown in Figure 1.

FIGURE 4 is an enlarged cross-sectional view of a portion of the catheter encircled by the line 4-4 of Figure 3.

FIGURE 5 is a side elevational view with portions broken away of the portion of the catheter in Figure 3 encircled by the line 5-5 of Figure 3.

FIGURE 6 is an enlarged cross-sectional view taken along the line 6-6 of Figure 3.

FIGURE 7 is a cross-sectional view of an alternative tip construction for the catheter.

## Detailed Description

In general, the guiding catheter assembly is adapted to be inserted into a vessel in the human body and consists of a first guiding catheter and a second or subselective guiding catheter. The second guiding catheter is removably disposed within the first guiding catheter and has a length so that it extends beyond the first guiding catheter. Each of the first and second guiding catheters includes a flexible elongated tubular member having a bore extending therethrough and having a hub secured to the proximal extremity of the tubular member. The bore in the tubular member of the first guiding catheter has a size so that the distal extremity of the guiding catheter can be inserted through the hub and into the bore of the tubular member of the first guiding catheter. The tubular member of the first guiding catheter is comprised solely of a braided jacket formed of an aromatic polyamide filament, the braided jacket having a cured plastic material impregnated therein to provide a smooth inner surface defining the bore of the first tubular member.

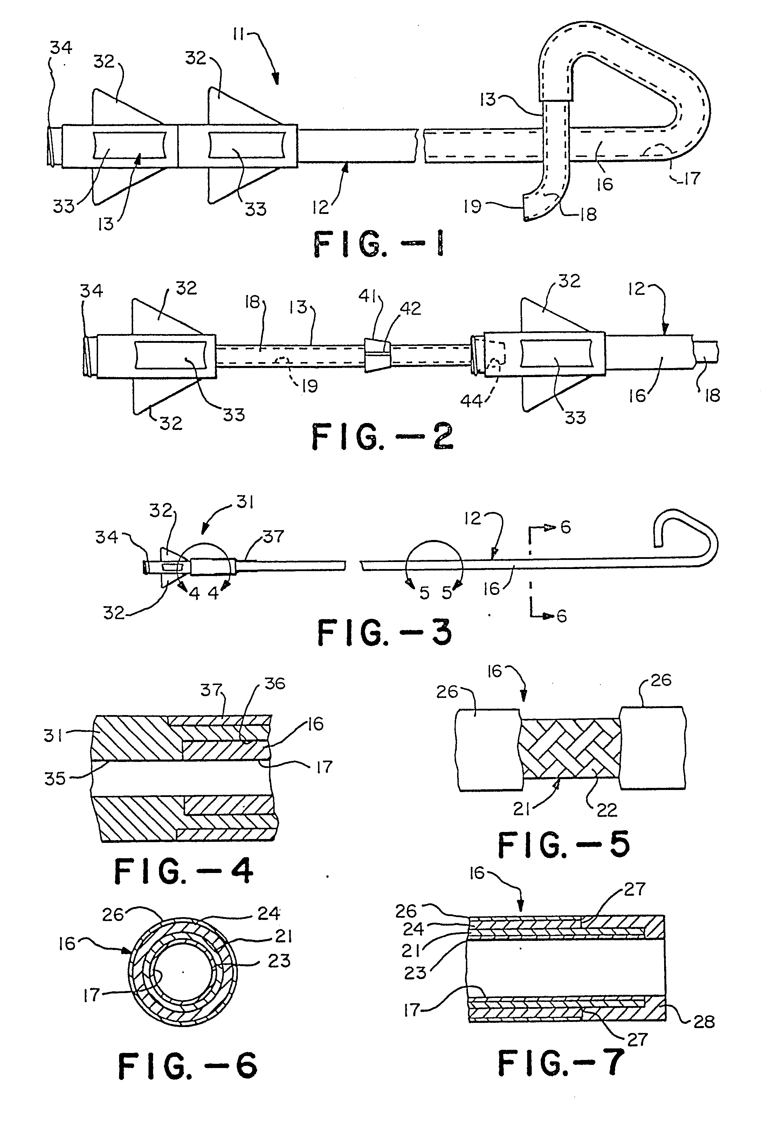

More in particular as shown in the drawings, the guiding catheter assembly 11 consists of a first or outer guiding catheter 12 and a second or inner guiding catheter 13. The second or inner guiding catheter is adapted to be positioned within the first or outer guiding catheter so that it is removably disposed therein so that it can serve as a subselective guiding catheter. As can be seen from Figure 1, the second or subselective guiding catheter 13 has a length which is greater than the length of the first guiding catheter 12 so that it will extend beyond the distal extremity of the outer guiding catheter.

Each of the first and second guiding catheters 12 and 13 is comprised of a flexible elongate tubular member having a bore extending therethrough. Thus, the first or outer guiding catheter 12 is provided with a flexible elongate tubular member 16 which is provided with a bore 17 extending therethrough. Similarly, the second guiding catheter 13 is comprised of a flexible elongate tubular member 18 having a bore 19 extending therethrough.

Each of the tubular members 16 and 18 for the first and second guide catheters 12 and 13 is manufactured in a similar manner. By way of example, the method of manufacture for the tubular member 16 will be described. A cylindrical elongate mandrel (not shown) which is circular in cross-section and of a suitable length is provided. The mandrel is formed of a suitable material such as Teflon so that it has a smooth outer surface. It also has a suitable outside diameter as, for example, for a 8 French guiding catheter the outside diameter of the mandrel would be .080 inches. A jacket 21 of high tensile strength fibers is braided onto the mandrel along the length thereof. The fibers are formed of a high tensile strength material of a suitable type such as Kevlar 49 Aramid yarn supplied by DuPont having a size or fineness ranging from 195 to 2130 denier. The tensile strength is in the order of $2.8 \times 10^9$Pa (400,000/lb per in²). It has a high modulus of $1.2 \times 10^{11}$Pa (18,000,000/lb in²). The yarns are comprised of multiple around cross-section continuous filaments of approximately 1.5 denier per filament. The yarns are formed into ribbons 22. The ribbons can have a suitable dimension depending upon the denier of the kevlar yarns utilized. By way of example, if a Kevlar yarn having a 380 denier is utilized, a typical ribbon has a cross-section dimension of approximately $.06 \times 1.3$ mm (2.5 × 50 mils). The jacket 21 is formed by utilizing a four × four braiding technique which means that eight different ribbons of Kevlar are braided onto the mandrel. A braiding pattern such as the one shown in Figure 5 can be utilized.

After the jacket 21 has been formed on the mandrel, a suitable adhesive such as a polyurethane adhesive is placed on the braided jacket 21 to saturate the jacket so that it extends through the ribbons 22 and forms a smooth coat onto the Teflon mandrel to form a thin internal layer 23 over the Teflon mandrel and interior of the braided jacket 21 (see Figure 6). At the same time an exterior layer 24 is also formed by the same polyurethane adhesive and is provided on the exterior of the impregnated jacket 21 to provide a smooth outer surface. The polyurethane adhesive is allowed to cure and thereafter, the outside diameter of the layer 24 is centerless ground to the appropriate size.

For an 8 French guiding catheter, the impregnated polyurethane layer 23 forms a very thin layer of polyurethane as, for example, one having a thickness of approximately 0.025 mm (.001 inch).

The braided jacket 21 has a thickness of approximately 0.15 mm (.006 inch). The polyurethane layer 24 has a thickness of approximately 0.1 mm (.004 inch). The outside diameter of the layer 24 when centerless ground is ground to a diameter of approximately 0.26 mm (.0103 inch). After the layer 24 has been formed and ground, another very thin layer 26 of a suitable thickness such as 0.025 mm (.001 inch) is sprayed onto the exterior of the layer 24 to provide an 8 French catheter which has an outside diameter of approximately 0.26 mm (.0105 inch). If desired, this outer thin layer 26 can be provided with a suitable color, as for example, black to provide a very shiny black outer appearance to the guiding catheter.

In order to make at least a portion of the guiding catheter visible under X-rays, a suitable radiopaque powder such as tantalum powder is incorporated in the distal extremity of the layer 24. By way of example, the last 4 inches of the distal extremity of the guiding catheter 12 can be impregnated with such a powder so that the bends provided in the distal extremity of the guiding catheter as shown in Figures 1 and 2 are substantially opaque to X-rays.

In order to provide a soft tip on the guide catheter, it is desirable to leave the last one millimeter of the distal extremity unsupported by the braided jacket 21. Therefore, it is desirable to form the layers 24 and 26 so they extend slightly beyond the braided jacket 21. With such a soft tip the catheter can be introduced into a vessel in the human body without danger of damaging the vessel such as could occur with a rigid or relatively hard tip.

Alternatively, if desired, a soft tip can be provided on the distal extremity of the catheter 12 in the manner shown in Figure 7. This can be accomplished by grinding an annular recess 27 onto the distal extremity to remove a portion of the layer 24. A soft plastic tip 28 formed of a suitable soft plastic is molded onto the distal extremity of the braided jacket 21.

After the tubular member 16 has been formed on the Teflon mandrel hereinbefore described, the mandrel can be readily removed from the tubular member by withdrawing the same to provide a very smooth inner surface for the lumen 17 formed by the thin layer 23 extending therethrough. Thereafter, the distal extremity of the tubular member 16 is formed into the desired shape as, for example, the conventional J-shape, which is shown in Figure 3 of the drawings by the application of heat in an appropriate mold.

A Luer-type fitting or hub 31 formed of a suitable material such as plastic is mounted on the proximal extremity of the tubular member 16. The fitting 31 is provided with a pair of triangular shaped wings 32 which extend diametrically of the fitting. The fitting 31 is also provided with a pair of flats 33 which are offset by approximately 90 degrees from the wings 32. The flats can be utilized for placing an appropriate trademark or other designation (not shown). A Luer-type connector 34 is provided on the proximal extremity of and integral with the fitting 31.

A cylindrical recess 36 (see Figure 4) is provided in the distal extremity of the fitting 31 and receives the proximal extremity of the tubular member 16 which is retained therein by suitable means such as an adhesive. The fitting 31 is provided with a bore 35 which is aligned with the lumen 17. Additional tubing 37 is provided which overlies the distal extremity of the fitting 31 and the proximal extremity of the flexible elongate tubular member 16. If desired, the tubing 37 can be formed of a heat shrinkable plastic so that it forms a tight fit on the flexible elongate member 16 and the fitting 31.

The second or subselective catheter is formed in the same manner as the first or outer guiding catheter 12 with the exception that it is smaller in diameter and wall thickness. As can be appreciated, the outside diameter of the tubular member 18 forming a part of the second or inner guiding catheter must have a size which is less than the inside diameter of the bore 17 of the tubular member 16. Thus, a subselective catheter 13 made in accordance with the present invention can have an outside diameter of 1.75 mm (.070 inch) so that it can be introduced through the 2mm (.080 inch) diameter of the bore 17 of an 8 French outer guiding catheter 12. The distal extremity of the subselective catheter 13 can be provided with different types of bends. The subselective catheter 13 would have a bore of approximately 1.25 mm (.050 inch) so that balloon dilatation catheters of various sizes can be accommodated. The distal extremity can be formed in a suitable manner such as by heating the same in a mold. With a subselective catheter 13 it is possible to reach lesions which are more distal from the ostium, particularly where it is necessary for the dilatation catheter to follow many curves.

After the fabrication steps heretofore described for the guiding catheters 12 and 13 shown in Figures 1 and 3 have been completed, it is desirable to coat the interior bore of the tubular member 16 and the exterior surface of the outer layer 28 with a suitable cross-linkable silicon based lubricant. This can be accomplished by applying the lubricant as a liquid to the interior of the bore and also to the exterior of the tubular member and permitting it to dry at room temperature or by heating the same.

In certain applications of the guiding assembly 11 it may be desirable to provide a plastic fitting 41 which is provided with a slit 42 extending longitudinally of the same, so that it can be slipped over the tubular member 18 of the second or subselective guiding catheter. This fitting 41 can be shaped in such a manner so that it can be slipped into the interior bore 44 of the Luer type fitting 31 provided on the outer guiding catheter 12. In this way, it is possible to provide a compression fitting to keep blood from oozing out between the outer guiding catheter 12 and the inner guiding catheter 13 and also to lock or clamp the subselective catheter 13 in a predetermined position with respect to the main or outer guiding catheter 12.

Operation and use of the guiding catheter assembly 11 and the guiding catheters 12 and 13 forming a part thereof may now be briefly described as follows. The first or outer guiding catheter 12 can be utilized in a conventional manner, such as that described in U.S. Patent No. 4,323,071 for precutaneous transluminal coronary angioplasty. Use of guiding catheters of the present invention in such a procedure is particularly advantageous because for the same size outside diameter guiding catheter a larger inside diameter bore is obtained. This larger inner bore makes it easier to perform dye injuctions. It also makes it possible to utilize larger size balloon dilatation catheters. It is possible to utilize smaller guiding catheters as, for example, a 7 French guiding catheter and obtain an inside diameter of a conventional guiding catheter, which makes it possible to utilize most balloon dilatation catheters presently on the market.

The use of such smaller guiding catheters makes it possible to traverse smaller vessels in the human body. In addition, the guiding catheter has other advantages. For example, because of the inclusion of tantalum powder its distal extremity can be readily viewed during the angioplasty procedure. Similarly, because of its construction the catheter has very great slickness on the inner and outer surfaces of the same which facilitates insertion and removal of the guiding catheter and also insertion and removal of guide wires and balloon dilatation catheters. In addition, because of its construction, the guiding catheter has higher torque values which greatly facilitates negotiating bends encountered in the vessels in the human body. These higher torque values are due to the composite polyurethane layers and the braided Kevlar ribbon jacket construction which is utilized in the guiding catheter.

When a particularly difficult vessel is encountered, it may be desirable to utilize a subselective guiding catheter such as the second or inner guiding catheter 13 shown in Figure 6. The distal extremity of this guiding catheter can be straightened out and inserted into the proximal extremity of the fitting 31 or to the catheter 12 and then introduced into the bore 17 of the catheter 12 and advanced in the bore 17 so that the distal extremity of the subselective or inner catheter 13 protrudes from the distal extremity of the first or outer guiding catheter 12. After the distal extremity of the subselective catheter 13 has been advanced beyond the distal extremity of the first guiding catheter 12 it assumes its conventional bend which can be observed by the use of floroscopy. By utilizing the subselective guiding catheter 13 it is possible to subselect arteries or branches of arteries which would be difficult, if not impossible, to negotiate with a standard catheter 12. The subselective catheter 13 can be readily advanced to apply a larger pushing force on the distal extremity of the subselective catheter. This is made possible because the outer guiding catheter 12 through which the subselective catheter 13 is advanced serves to prevent buckling or kinking of the subselective catheter 13 and therefore, increases its column strength. Because of this feature, it is possible to utilize a subselective catheter 13 having a thinner wall thereby also making it possible to provide smaller subselective catheters.

After the outer guiding catheter 12 is in position, a small diameter guidewire 46 of a conventional type can be introduced through the bore 17 and advanced into the desired vessel or stenosis. After the guidewire 46 is in place, the subselective catheter can be introduced over the guidewire 46 and introduced into the bore 17 until its distal extremity extends beyond the distal extremity of the main guiding catheter 12. It is also advanced over the guidewire into or near the desired stenosis. Thereafter, a balloon dilatation catheter having a low profile can be introduced into the subselective guiding catheter and advanced over the guidewire 46 into the stenosis. The guidewire 46 then can be removed. The stenosis can then be dilated by inflating the balloon in the conventional manner. Thereafter, the balloon dilatation catheter, the guide wire 46 and the guiding catheters 12 and 13 can be removed.

The principle advantages of the subselective catheter 13 is that, in conjunction with an outer guiding catheter, it provides additional support to the balloon catheter while crossing a tight stenosis and/or a stenosis deep in a tortuous vessel.

The subselective catheter would most likely be used in conjunction with an 1F guiding catheter, (ID = 2.3mm) (ID = .092 inch). This would allow the subselective catheter to have an OD of 2 mm (.080 inch) and an ID of 1.5 mm (.060 inch). The subselective catheter would then accommodate most small profile balloon catheters. From the foregoing can be seen that the guiding catheters of the present invention in and of themselves have numerous advantages over presently available guiding catheters. In addition, the guiding catheters are formed in such a manner so that the use of subselective guiding catheters is made possible. This makes it possible to treat arterial vessels which are very small in size and difficult to reach.

## Claims

1. In a guiding catheter assembly adapted to be inserted into a vessel, a first guiding catheter and a second guiding catheter removably disposed within the first guiding catheter and having a length so that it extends beyond the distal extremity of the first guiding catheter, each of said first and second guiding catheters including a flexible elongate tubular member having a bore extending therethrough and a fitting secured to the proximal extremity of the tubular member, the bore in the tubular member being of a size so that the portion of the second guiding catheter extending beyond the fitting can be introduced into the bore of the tubular member of the first guiding catheter.

2. An assembly as claimed in Claim 1 wherein said tubular member is comprised solely of braided ribbons having a cured plastic material impregnated therein providing a smooth inner surface defining the bore and additional cured plastic providing a smooth outer surface.

3. A guiding catheter as claimed in Claim 2 wherein said tubular member includes an outer plastic layer having an opaque coloring therein.

4. A guiding catheter as claimed in Claims 1, 2 or 3, wherein the tips of the flexible tubular members are relatively soft and are free of the braided ribbons.

5. A guiding catheter as claimed in Claim 2 wherein said braided ribbons are formed of Kevlar 49 aramid yarns.

6. A catheter assembly as claimed in Claims 1, 2, 3, 4 or 5 together with a hub mounted on the proximal end of each tubular member, and fitting carried by the second catheter and adapted to said seat in the hub provided on the proximal extremity of the outer guiding catheter to provide a liquid tight seal and also to retain the inner guiding catheter in a predetermined position with respect to the outer guiding catheter.

7. In a guiding catheter, a flexible tubular member having a bore extending therethrough, said tubular member being comprised of a jacket formed solely of braided ribbons having a cured plastic material impregnated therein and providing a smooth inner surface defining the bore.

8. A catheter as claimed in Claim 7 wherein said plastic material is polyurethane.

9. A catheter as claimed in Claims 7 or 8, together with additional layers of plastic formed on said braided jacket and wherein one of said layers includes an opaque coloring.

10. A catheter as claimed in Claims 7, 8 or 9, wherein the distal extremity of the tubular members has a radiopaque powder incorporated in same.

11. A catheter as claimed in Claim 10 wherein radiopaque powder includes tantalum.

12. A catheter as claimed in Claims 7, 8, 9, 10 or 11, together with at least one additional layer 4 plastic formed on said jacket, said layer being centerless ground to a precise diameter.

13. A catheter as in Claim 1 wherein each of the first and second guiding catheters is provided with a soft tip.

14. In a method for making a guiding catheter, providing a mandrel formed of a material having a smooth outer surface, braiding a jacket of ribbons comprised of yarn onto said mandrel, impregnating said ribbons with a plastic material and curing the same so that a smooth inner surface is provided engaging the mandrel, removing the mandrel so that there is provided a flexible tubular member which is formed solely of the braided ribbons having a cured plastic material impregnated therein and thereon.

15. A method as claimed in Claim 14 together with the step of applying additional layers of plastic onto the braided jacket.

16. A method as claimed in Claim 15 together with the step of adding an opaque coloring to one of the layers of plastic material.

17. A method as claimed in Claim 14 together with the step of impregnating the distal extremity of at least one of the plastic layers with a radiopaque powder.

18. A method as claimed in Claim 14 together with the step of placing a predetermined bend in the distal extremity of the flexible tubular member.

19. A method as claimed in Claim 15 together with the step of centerless grinding at least one of the layers of plastic material.

FIG. — 1

FIG. — 2

FIG. — 3

FIG. — 4

FIG. — 5

FIG. — 6

FIG. — 7

| | DOCUMENTS CONSIDERED TO BE RELEVANT | | EP 87119364.5 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,Y | US - A - 4 323 071 (J.B.SIMPSON et al.) | 1,6 | A 61 M 25/00 |
| A | * Fig. 1,2; column 3, lines 43-55; claims 1,6 * | 13 | |
| | -- | | |
| Y | GB - A - 2 118 840 (H.G.WALLACE) | 1,6 | |
| | * Fig. 1,2; page 1, line 125 - page 2, line 17 * | | |
| | -- | | |
| Y | US - A - 4 516 972 (W.J.SAMSON) | 7,8 | |
| A | * Totality; especially column 2, lines 9-44; column 3, lines 1-26; column 4, lines 40-47; abstract; claims 1,3 * | 2,4,5, 13,14 | |
| | -- | | |
| Y | DE - A1 - 3 019 995 (INST. F.TEXTIL) | 7,8 | |
| A | * Fig. 1,2; claims 1,6,10,11; page 7, last paragraph - page 8, line 1; page 11, 4th paragraph - page 12, 1st paragraph * | 14 | **TECHNICAL FIELDS SEARCHED (Int. Cl.4)** <br> A 61 M 25/00 |
| | -- | | |
| A | US - A - 4 425 919 (W.ALSTON et al.) | 2,7,14 | |
| | * Totality; especially fig. 1,3A, 5; column 5, lines 32-38; claims 1,8 * | | |
| | -- | | |
| A | GB - A - 1 511 269 (SHERWOOD MED.) | 3,9,10 | |
| | * Fig. 2; page 2, lines 100-108; page 3, lines 4-10,27-30,37-40 * | | |
| | ---- | | |

The present search report has been drawn up for all claims

| Place of search | Date of completion of the search | Examiner |
|---|---|---|
| VIENNA | 24-05-1988 | LUDWIG |